# EUROPEAN PATENT SPECIFICATION

(11) **EP 1 812 797 B1**
(45) Date of publication and mention of the grant of the patent: **20.03.2013**
(21) Application number: 05818382.3
(22) Date of filing: 28.10.2005
(51) Int. Cl.: C07K 16/30, G01N 33/574, A61K 38/17, A61K 39/395, A61P 35/00, A61P 9/00, A61P 27/00, A61K 31/337, C07K 16/18

(54) **COMPOSITIONS AND METHODS FOR THE TREATMENT AND PREVENTION OF HYPERPROLIFERATIVE DISEASES**
ZUSAMMENSETZUNGEN UND VERFAHREN ZUR BEHANDLUNG UND VORBEUGUNG VON HYPERPROLIFERATIONSKRANKHEITEN
COMPOSITIONS ET METHODES PERMETTANT DE TRAITER ET DE PREVENIR DES MALADIES HYPERPROLIFERATIVES

(30) Priority: 28.10.2004 US 623197 P
(43) Date of publication of application: 01.08.2007
(73) Proprietor: Lpath, Inc., San Diego, CA 92121 (US)
(72) Inventor: SABBADINI, Roger, A., Lakeside, CA 92040 (US); CAVALLI, Amy, L., San Diego, CA 92117 (US); GARLAND, William, San Clemente, CA 92672 (US)
(74) Representative: Schiweck, Weinzierl & Koch
(86) International application number: PCT/US2005/039303
(87) International publication number: WO 2006/078336

(56) References cited:
- WO-A-02/17899
- WO-A-99/16888
- WO-A-02/051439
- WO-A1-03/097028
- US-A- 5 877 167
- HLA TIMOTHY: "Physiological and pathological actions of sphingosine 1-phosphate." SEMINARS IN CELL & DEVELOPMENTAL BIOLOGY OCT 2004, vol. 15, no. 5, 1 October 2004 (2004-10-01), pages 513-520, XP002455394 ISSN: 1084-9521
- SPIEGEL S ET AL: "Sphingosine 1-phosphate as a therapeutic agent." LEUKEMIA : OFFICIAL JOURNAL OF THE LEUKEMIA SOCIETY OF AMERICA, LEUKEMIA RESEARCH FUND, U.K SEP 2002, vol. 16, no. 9, September 2002 (2002-09), pages 1596-1602, XP002455395 ISSN: 0887-6924
- WANG F ET AL: "Sphingosine-1-phosphate inhibits motility of human breast cancer cells independently of cell surface receptors." CANCER RESEARCH 15 DEC 1999, vol. 59, no. 24, 15 December 1999 (1999-12-15), pages 6185-6191, XP002455663 ISSN: 0008-5472
- CUVILLIER O ET AL: "SPHINGOSINE 1-PHOSPHATE ANTAGONIZES APOPTOSIS OF HUMAN LEUKEMIA CELLS BY INHIBITING RELEASE OF CYTOCHROME C AND SMAC/DIABLO FROM MITOCHONDRIA" BLOOD, W.B.SAUNDERS COMPANY, ORLANDO, FL, US, vol. 98, no. 9, 1 November 2001 (2001-11-01), pages 2828-2836, XP008043224 ISSN: 0006-4971
- OGRETMEN BESIM ET AL: "Biologically active sphingolipids in cancer pathogenesis and treatment." NATURE REVIEWS. CANCER AUG 2004, vol. 4, no. 8, August 2004 (2004-08), pages 604-616, XP002455664 ISSN: 1474-175X
- GOETZL EDWARD J ET AL: "An IgM-kappa rat monoclonal antibody specific for the type 1 sphingosine 1-phosphate G protein-coupled receptor with antagonist and agonist activities." IMMUNOLOGY LETTERS 30 APR 2004, vol. 93, no. 1, 30 April 2004 (2004-04-30), pages 63-69, XP002455665 ISSN: 0165-2478
- VISENTIN BARBARA ET AL: "Validation of an anti-sphingosine-1-phosphate antibody as a potential therapeutic in reducing growth, invasion, and angiogenesis in multiple tumor lineages." CANCER CELL MAR 2006, vol. 9, no. 3, March 2006 (2006-03), pages 225-238, XP002455666 ISSN: 1535-6108
- DAVAILLE J ET AL: "ANTIPROLIFERATIVE PROPERTIES OF SPHINGOSINE 1-PHOSPHATE IN HUMAN HEPATIC MYOFIBROBLASTS A CYCLOOXYGENASE-2 MEDIATED PATHWAY" JOURNAL OF BIOLOGICAL CHEMISTRY, AMERICAN SOCIETY OF BIOLOCHEMICAL BIOLOGISTS, BIRMINGHAM,, US, vol. 275, no. 44, 3 November 2000 (2000-11-03), pages 34628-34633, XP002945852 ISSN: 0021-9258
- VEKICH JOHN A ET AL: "Tumorigenic and angiogenic effects of S1P are mitigated by an anti-S1P mAb in multiple murine models of cancer." PROCEEDINGS OF THE AMERICAN ASSOCIATION FOR CANCER RESEARCH ANNUAL MEETING, vol. 46, April 2005 (2005-04), page 557, XP002455667 & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X

## Description

### TECHNICAL FIELD

The invention relates generally to the area of treatment and/or prevention of hyperproliferative diseases and disorders and, in particular, cancer and other pathologies characterized by excessive neovascularization. These useful results are achieved by the use of agents, and compositions that contain such agents that interfere with the production and/or biological activities of sphingolipids and their metabolites.

### BACKGROUND OF THE INVENTION

### 1. Introduction.

The following description includes information that may be useful in understanding the present invention. It is not an admission that any such information is prior art, or relevant, to the presently claimed inventions, or that any publication specifically or implicitly referenced is prior art.

### 2. Background.

There are many known hyperproliferative disorders, in which cells of various tissues and organs exhibit aberrant patterns of growth, proliferation, migration, signaling, senescence, and death. While a number of treatments have been developed to address some of these diseases, many still remain largely untreatable with existing technologies, while in other cases, while treatments are available, they are frequently less than optimal and are seldom curative.

Cancer represents perhaps the most widely recognized class of hyperproliferative disorders. Cancers are a devastating class of diseases, and together, they have a mortality rate second only to cardiovascular disease. Many cancers are not fully understood on a molecular level. As a result, cancer is a major focus of research and development programs for both the United States government and pharmaceutical companies. The result has been an unprecedented R&D effort and the production of many valuable therapeutic agents to help in the fight against cancer.

Unfortunately the enormous amount of cancer research has not been enough to overcome the significant damage caused by cancer. There are still over one million new cases of cancer diagnosed annually and over five hundred thousand deaths in the United States alone. This is a dramatic demonstration that even though an enormous effort has been put forth to discover new therapeutics for cancer, effective therapeutic agents to combat the disease remain elusive.

Cancer is now primarily treated with one or a combination of three types of therapies, surgery, radiation, and chemotherapy. Surgery involves the bulk removal of diseased tissue. While surgery is sometimes effective in removing tumors located at certain sites, for example, in the breast, colon, and skin, it cannot be used in the treatment of tumors located in other areas, such as the backbone, nor in the treatment of disseminated neoplastic conditions such as leukemia. Radiation therapy involves the exposure of living tissue to ionizing radiation causing death or damage to the exposed cells. Side effects from radiation therapy may be acute and temporary, while others may be irreversible. Chemotherapy involves the disruption of cell replication or cell metabolism.

Further insult is that current therapeutic agents usually involve significant drawbacks for the patient in the form of toxicity and severe side effects. Therefore, many groups have recently begun to look for new approaches to fighting the war against cancer. These new so-called "innovative therapies" include gene therapy and therapeutic proteins such as monoclonal antibodies.

The first monoclonal used in the clinic for the treatment of cancer was Rituxan (rituximab) which was launched in 1997, and has demonstrated the utility of biospecific monoclonal antibodies as therapeutic agents. Thus, not surprisingly, sixteen other monoclonal antibodies have since been approved for use in the clinic, including six that are prescribed for cancer. The success of these products, as well as the reduced cost and time to develop monoclonal antibodies as compared with small molecules has made monoclonal antibody therapeutics the second largest category of drug candidates behind small molecules. Further, the exquisite specificity of antibodies as compared to small molecule therapeutics has proven to be a major advantage both in terms of efficacy and toxicity. For cancer alone there are currently more than 270 industry antibody R&D projects with more than 50 companies involved in developing new cancer antibody therapeutics. Consequently, monoclonal antibodies are poised to become a major player in the treatment of cancer and they are estimated to capture an increasing share of the cancer therapeutic market.

The identification of extracellular mediators that promote tumor growth and survival is a critical step in discovering therapeutic interventions that will reduce the morbidity and mortality of cancer. As described below, sphingosine-1-phosphate (S1P), a key component of sphingolipid signaling cascade, is considered to be a pleiotropic, tumorigenic growth factor. SIP promotes tumor growth by stimulating cell proliferation, cell survival, and metastasis. S1P also promotes tumor angiogenesis by supporting the migration and survival of endothelial cells as they form new vessels within tumors. Taken together, S1P initiates a proliferative, pro-angiogenic, and anti-apoptotic sequence of events contributing to cancer progression. Thus, therapies that modulate, and, in particular, reduce S1P levels *in vivo* will be effective in the treatment of cancer.

Ogretmen et al. (Nature Reviews 2004, 4(8):604-16) relates to the role of sphingolipids in cancer development and progression. Based on the finding that sphingosine-1-phosphate (S1P) is tumorigenic, the authors hypothesize that antagonizing S1P may have use in cancer therapy.

WO 02051439 discloses the use of antibodies to reduce circulating concentrations of S1P for the treatment of cardiovascular diseases.

### 3. Definitions.

Before describing the instant invention in detail, several terms used in the context of the present invention will be defined. In addition to these terms, others are defined elsewhere in the specification, as necessary. Unless otherwise expressly defined herein, terms of art used in this specification will have their art-recognized meanings.

An "anti-S1P molecule" refers to any molecule that interferes with S1P activity, particularly an SIP activity on cells that are, or are capable of, proliferating. Representative examples of such molecules include anti-S1P antibodies, fragments from anti-S1P antibodies capable of specifically interacting with S1P, and agents that comprising a first binding moiety and a second binding moiety, wherein one of the binding moieties is specifically reactive with SIP.

The term "chemotherapeutic agent" means anti-cancer and other anti-hyperproliferative agents. Put simply, a "chemotherapeutic agent" refers to a chemical intended to destroy cells and tissues. Such agents include, but are not limited to: (1) DNA damaging agents and agents that inhibit DNA synthesis: anthracyclines (doxorubicin, donorubicin, epirubicin), alkylating agents (Cyclophosphamide, Mitomycin C, chemical mustards), platinum derivatives (cisplatin, carboplatin, cis diamminedichloroplatinum), telomerase and topoisomerase inhibitors (Camptosar), (2) tubulin-depolymerizing agents: taxoids (paclitaxel, docetaxel, BAY 59-8862), (3) anti-metabolites: fluorinated pyrimidines (5-FU, capecitabine, 5-DFUR, gemcitabine), proteosome inhibitors (Velcade), methotrexates, (4) anti-angiogenics (Avastin, thalidomide), vascular disrupting agents (flavonoids/flavones, DMXAA), combretastatin derivatives (CA4DP, ZD6126, AVE8062A), (5) biologics such as antibodies (Herceptin, Avastin, Panorex, Rituxin, Zevalin, Mylotarg, Campath, Bexxar, Erbitux), and (6) endocrine therapy: aromatase inhibitors (4-hydroandrostendione, exemestane, aminoglutehimide, anastrzole, letozole), anti-estrogens (Tamoxifen, Toremifine, Raoxifene, Faslodex), steroids such as dexamethasone, (7) Immunomodulators: cytokines such as IFN-beta and IL2), inhibitors to integrins, other adhesion proteins and matrix metalloproteinases), (8) histone deacetylase inhibitors, (9) inhibitors of signal transduction such as inhibitors of tyrosine kinases like gleevec, (10) inhibitors of heat shock proteins, (11) retinoids such as all trans retinoic acid and (12) inhibitors of growth factor recptors or the growth factors themselves.

One class of chemotherapeutic agents are alkylating agents. An "alkylating agent" refers to a chemotherapeutic compound that chemically modify DNA and disrupt its function. Some alkylating agents alkylate DNA, others cause formation of cross links between nucleotides on the same strand, or the complementary strand, of a double-stranded DNA molecule, while still others cause base-pair mismatching between DNA strands. Exemplary alkylating agents include bendamustine, busulfan, carboplatin, carmustine, cisplatin, chlorambucil, cyclophosphamide, dacarbazine, hexamethylmelamine, ifosphamide, lomustine, mechlorethamine, melphalan, mitotane, mytomycin, pipobroman, procarbazine, streptozocin, thiotepa, and triethylenemelamine. Another class of chemotherapeutic agents is the anti-metabolites. An "anti-metabolite" refers to a chemotherapeutic agent that interferes with the synthesis of biomolecules, including those required for DNA synthesis (*e.g*., nucleosides and nucleotides) needed to synthesize DNA. Examples of anti-metabolites include capecitabine, chlorodeoxyadenosine, cytarabine (and its activated form, ara-CMP), cytosine arabinoside, dacabazine, floxuridine, fludarabine, 5-fluorouracil, gemcitabine, hydroxyurea, 6-mercaptopurine, methotrexate, pentostatin, trimetrexate, and 6-thioguanine. An "anti-mitotic" chemotherapeutic agent refers to a chemotherapeutic agent that interferes with mitosis, typically through disruption of microtubule formation. Examples of anti-mitotic compounds include navelbine, paclitaxel, taxotere, vinblastine, vincristine, vindesine, and vinorelbine. An "intercalating agent" refers to a chemotherapeutic agent that inserts itself between adjacent base pairs in a double-stranded DNA molecule, disrupting DNA structure and interfering with DNA replication, gene transcription, and/or the binding of DNA binding proteins to DNA.

The term "combination therapy" refers to a therapeutic regimen that involves the provision of at least two distinct therapies to achieve an indicated therapeutic effect. For example, a combination therapy may involve the administration of two or more chemically .distinct active ingredients, for example, a fast-acting chemotherapeutic agent and an anti-S I P antibody. Alternatively, a combination therapy may involve the administration of an anti-S1P molecule (e.g., an anti-S 1 P antibody) and/or one or more chemotherapeutic agents, alone or together with the delivery of radiation therapy and/or surgery. In the context of the administration of two or more chemically distinct active ingredients, it is understood that the active ingredients may be administered as part of the same composition or as different compositions. When administered as separate compositions, the compositions comprising the different active ingredients may be administered at the same or different times, by the same or different routes, using the same of different dosing regimens, all as the particular context requires and as determined by the attending physician. Similarly, when one or more anti-S 1 P molecule species, alone or in conjunction with a chemotherapeutic agents are combined with, for example, radiation and/or surgery, the drug(s) may be delivered before or after surgery or radiation treatment.

The term "hyperproliferative disorder" refers to diseases and disorders associated with, the uncontrolled proliferation cells, including but not limited to uncontrolled growth of organ and tissue cells resulting in cancers and benign tumors. Hyperproliferative disorders associated with endothelial cells can result in diseases of angiogenesis such as angiomas, endometriosis, obesity, Age-related Macular Degeneration and various retinopaties, as well as the proliferation of ECs and smooth muscle cells that cause restenosis as a consequence of stenting in the treatment of atherosclerosis. Hyperproliferative disorders involving fibroblasts (i.e., fibrogenesis) include but are not limited to disorers of excessive scaring (i.e., fibrosis) such as Age-related Macular Degeneration, cardiac remodeling and failure associated with myocardial infarction, excessive wound healing such as commonly occurs as a consequence of surgery or injury, keloids, and fibroid tumors and stenting.

In the context of this invention, a "liquid composition" refers to one that, in its filled and finished form as provided from a manufacturer to an end user (e.g., a doctor or nurse), is a liquid or solution, as opposed to a solid. Here, "solid" refers to compositions that are not liquids or solutions. For example, solids include dried compositions prepared by lyophilization, freeze-drying, precipitation, and similar procedures.

"Monotherapy" refers to a treatment regimen based on the delivery of one therapeutically effective compound, whether administered as a single dose or several doses over time.

"Neoplasia" refers to abnormal and uncontrolled cell growth. A "neoplasm", or tumor, is an abnormal, unregulated, and disorganized proliferation of cell growth, and is generally referred to as cancer. A neoplasm may be benign or malignant. A neoplasm is malignant, or cancerous, if it has properties of destructive growth, invasiveness, and metastasis. Invasiveness refers to the local spread of a neoplasm by infiltration or destruction of surrounding tissue, typically breaking through the basal laminas that define the boundaries of the tissues, thereby often entering the body's circulatory system. Metastasis typically refers to the dissemination of tumor cells by lymphatics or blood vessels. Metastasis also refers to the migration of tumor cells by direct extension through serous cavities, or subarachnoid or other spaces. Through the process of metastasis, tumor cell migration to other areas of the body establishes neoplasms in areas away from the site of initial appearance

A "patentable" composition, process, machine, or article of manufacture according to the invention means that the subject matter satisfies all statutory requirements for patentability at the time the analysis is performed. For example, with regard to novelty, non-obviousness, or the like, if later investigation reveals that one or more claims encompass one or more embodiments that would negate novelty, non-obviousness, *etc.,* the claim(s), being limited by definition to "patentable" embodiments, specifically exclude the unpatentable embodiment(s). Also, the claims appended hereto are to be interpreted both to provide the broadest reasonable scope, as well as to preserve their validity. Furthermore, if one or more of the statutory requirements for patentability are amended or if the standards change for assessing whether a particular statutory requirement for patentability is satisfied from the time this application is filed or issues as a patent to a time the validity of one or more of the appended claims is questioned, the claims are to be interpreted in a way that (1) preserves their validity and (2) provides the broadest reasonable interpretation under the circumstances.

The term "pharmaceutically acceptable salt" refers to salts which retain the biological effectiveness and properties of the agents and compounds of this invention and which are not biologically or otherwise undesirable. In many cases, the agents and compounds of this invention are capable of forming acid and/or base salts by virtue of the presence of charged groups, for example, charged amino and/or carboxyl groups or groups similar thereto. Pharmaceutically acceptable acid addition salts may be prepared from inorganic and organic acids, while pharmaceutically acceptable base addition salts can be prepared from inorganic and organic bases. For a review of pharmaceutically acceptable salts see Berge, et al. ((1977) J. Pharm. Sci., vol. 66, 1).

A "plurality" means more than one.

The terms "separated", "purified", "isolated", and the like mean that one or more components of a sample contained in a sample-holding vessel are or have been physically removed from, or diluted in the presence of, one or more other sample components present in the vessel. Sample components that may be removed or diluted during a separating or purifying step include, chemical reaction products, unreacted chemicals, proteins, carbohydrates, lipids, and unbound molecules.

The term "species" is used herein in various contexts, *e.g*., a particular species of chemotherapeutic agent. In each context, the term refers to a population of chemically indistinct molecules of the sort referred in the particular context.

"Specifically associate", "specific association," and the like refer to a specific, non-random interaction between two molecules, which interaction depends on the presence of structural, hydrophobic/hydrophilic, and/or electrostatic features that allow appropriate chemical or molecular interactions between the molecules.

Herein, "stable" refers to an interaction between two molecules (*e.g*., a peptide and a TLR molecule) that is sufficiently stable such that the molecules can be maintained for the desired purpose or manipulation. For example, a "stable" interaction between a peptide and a TLR molecule refers to one wherein the peptide becomes and remains associated with a TLR molecule for a period sufficient to achieve the desired effect.

A "subject" or "patient" refers to an animal in need of treatment that can be effected by molecules of the invention. Animals that can be treated in accordance with the invention include vertebrates, with mammals such as bovine, canine, equine, feline, ovine, porcine, and primate (including humans and non-humans primates) animals being particularly preferred examples.

A "therapeutically effective amount" (or "effective amount") refers to an amount of an active ingredient, *e.g*., an agent according to the invention, sufficient to effect treatment when administered to a subject in need of such treatment. Accordingly, what constitutes a therapeutically effective amount of a composition according to the invention may be readily determined by one of ordinary skill in the art. In the context of cancer therapy, a "therapeutically effective amount" is one that produces an objectively measured change in one or more parameters associated with cancer cell survival or metabolism, including an increase or decrease in the expression of one or more genes correlated with the particular cancer, reduction in tumor burden, cancer cell lysis, the detection of one or more cancer cell death markers in a biological sample (*e.g*., a biopsy and an aliquot of a bodily fluid such as whole blood, plasma, serum, urine, *etc*.), induction of induction apoptosis or other cell death pathways, *etc.* Of course, the therapeutically effective amount will vary depending upon the particular subject and condition being treated, the weight and age of the subject, the severity of the disease condition, the particular compound chosen, the dosing regimen to be followed, timing of administration, the manner of administration and the like, all of which can readily be determined by one of ordinary skill in the art. It will be appreciated that in the context of combination therapy, what constitutes a therapeutically effective amount of a particular active ingredient may differ from what constitutes a therapeutically effective amount of the active ingredient when administered as a monotherapy (*i.e.,* a therapeutic regimen that employs only one chemical entity as the active ingredient).

The term "treatment" or "treating" means any treatment of a disease or disorder, including preventing or protecting against the disease or disorder (that is, causing the clinical symptoms not to develop); inhibiting the disease or disorder (*i.e.,* arresting or suppressing the development of clinical symptoms; and/or relieving the disease or disorder (*i.e*., causing the regression of clinical symptoms). As will be appreciated, it is not always possible to distinguish between "preventing" and "suppressing" a disease or disorder since the ultimate inductive event or events may be unknown or latent. Accordingly, the term "prophylaxis" will be understood to constitute a type of "treatment" that encompasses both "preventing" and "suppressing". The term "protection" thus includes "prophylaxis".

The term "therapeutic regimen" means any treatment of a disease or disorder using chemotherapeutic and cytotoxic agents, radiation therapy, surgery, gene therapy, DNA vaccines and therapy, siRNA therapy, anti-angiogenic therapy, immunotherapy, bone marrow transplants, apatamers and other biologics such as antibodies and antibody variants, receptor decoys and other protein-based therapeutics.

According to the Merck Manual (14th edition, p. 1206) cancer is "a cellular malignancy whose unique characteristics - loss of normal controls - results in unregulated growth, lack of differentiation, and ability to invade local tissue and metastasize." Similarly, the National Cancer Institute of the NIH (see http://cancer.gov/) defines cancer as, "A term for diseases in which abnormal cells divide without control. Cancer cells can invade nearby tissues and can spread through the bloodstream and lymphatic system to other parts of the body." Cancer cells also avoid natural cell death and stimulate the formation of their own blood supply through a process known as angiogenesis. The NCI defines angiogenesis as "blood vessel formation. Tumor angiogenesis is the growth of blood vessels from surrounding tissue to a solid tumor. This is caused by the release of chemicals by the tumor." Inflammation is defined by the NIH as, "A response of redness, swelling, pain, and a feeling of heat in certain areas that is meant to protect tissues affected by injury or disease."

### SUMMARY OF THE INVENTION

One aspect of the invention concerns a monoclonal antibody that binds to and inhibits S1P for use in methods for treating a hyperproliferative disorder as claimed in the appended claims. These methods comprise administering to a mammal (e.g., a bovine, canine, equine, ovine, or porcine animal, particularly a human) known or suspected to suffer from an S 1P-associated hyperproliferative disorder a therapeutically effective amount of a composition comprising an agent that inhibits with S1P activity, preferably in a pharmaceutically or veterinarily acceptable carrier, as the intended application may require. S1P-associated hyperproliferative disorders include neoplasias, disorder associated with endothelial cell proliferation, and disorders associated with fibrogenesis. Most often, the neoplasia will be a cancer. Typical disorders associated with endothelial cell proliferation are angiogenesis-dependent disorders, for example, cancers caused by a solid tumors, hematological tumors, and age-related macular degeneration. Disorders associated with fibrogenesis include those than involve aberrant cardiac remodeling, such as cardiac failure.

The agent that interferes with S1P activity is a monoclonal antibody specifically reactive with S1P.

In a preferred embodiment, the composition comprising an agent that inhibits SIP activity is administered as a monotherapy, while in other preferred embodiments, the composition comprising the agent that inhibits S1P activity is administered as part a combination therapy. Preferred combination therapies include, in addition to administration of the composition comprising an agent that inhibits with S1P activity, delivering a second therapeutic regimen selected from the group consisting of administration of a chemotherapeutic agent, radiation therapy, surgery, and a combination of any of the foregoing.

Other features and advantages of the invention will be apparent from the following detailed description and appended claims.

As those in the art will appreciate, the following detailed description describes certain preferred embodiments of the invention in detail, and is thus only representative and does not depict the actual scope of the invention. Before describing the present invention in detail, it is understood that the invention is not limited to the particular aspects and embodiments described, as these may vary. It is also to be understood that the terminology used herein is for the purpose of describing particular embodiments only, and is not intended to limit the scope of the invention defined by the appended claims.

### DETAILED DESCRIPTION

The present invention is based on the surprising discovery that anti-S1P molecules, particularly anti-S1P antibodies, can be used to treat hyperproliferative diseases associated with SIP activity. Additionally, a class of anti-S1P molecules, namely, agents that comprise a first binding moiety and a second binding moiety, one of which moieties bind SIP, is also described.

### 1. Introduction.

### A. Sphingolipids

Sphingolipids are primary structural components of cell membranes that also serve as cellular signaling and regulatory molecules. The sphingolipid signaling cascade includes the bioactive lipid mediators, ceramide (CER), sphingosine (SPH), and sphingosine-1-phosphate (S1P). These mediators are derived from sphingomyelin, which is present in the plasma membranes of all mammalian cells.

The neutral form of sphingomyelinase (nSMase) is a key early components of the sphingolipid signaling pathway. Tumor necrosis factor alpha (TNFα) is a well-known activator of nSMase, CER production, and apoptosis in many cell types, including cancer cell lines, and the activation of nSMase has been shown to be critical for TNFα- induced apoptosis, making it a target for drug discovery.

The sphingolipid signaling molecule, S1P, is produced from SPH through the action of sphingosine kinase (SPHK). Two isoforms of the kinase have been identified, SPHK1 and SPHK2. While CER and SPH are commonly associated with apoptosis, S1P is typically viewed as an extracellular mediator of cell proliferation and activation of survival pathways. S1P can act as a ligand for a set of G Protein Coupled Receptors (GPCRs) belonging to the S1P/LPA receptor family, formerly known as Edg receptors; however, intracellular actions of S1P have also been suggested. Moreover, it has been suggested that the balance between CER/SPH levels versus S1P provides a rheostat mechanism that decides whether a cell is sent into the death pathway or is protected from apoptosis by S1P.

The key regulatory enzyme of the rheostat mechanism is SPHK, whose role is to convert the death-promoting sphingolipids (CER/SPH) into the growth-promoting S1P. It has been shown that NIH-3T3 fibroblasts stably transfected with SPHK exhibit enhanced cell proliferation accompanied by increased S1P production, and SPHK over-expressers can escape contact inhibition, a property commonly exhibited by transformed cells. Thus, S1P can enhance metastatic potential of selected human cancer cell lines. Moreover, the SPHK transfectants can produce tumors when injected subcutaneous into NOD/SCID mice. Significantly, SPHK is over-expressed in many solid tumors, such as those of the breast, colon, lung, ovary, stomach, uterus, kidney, and rectum. It has been shown that apoptosis can be induced in several human tumor-derived cell lines by treatment with a small molecule inhibitor of SPHK, which also reduce S1P levels. Also, genotoxics and other anti-neoplastics down-regulate SPHK as part of their mechanisms of action. Similarly, down-regulation of SPHK by siRNA can decrease melanoma cells resistance to apoptosis, while the protective effect of enhanced Bcl-2 expression has been attributed to increased SPHK expression. Further, the anti-neoplastic effect of FTY70 has been attributed to its down-regulation of S1P receptors, suggesting that interfering with S1P action at the receptor level could also be valuable in anti-tumor therapy, for example, through the use of an antibody that interferes with S1P receptor binding. Taken together, these findings demonstrate that S1P is a growth factor likely produced by tumor cells themselves, and that lowering the concentration of S1P may cause the apoptosis seen upon growth factor withdrawal.

### B. S1P as a Valid Target for Cancer Therapy.

One cancer therapy strategy is to reduce the biologically available extracellular levels of the tumor-promoter, S1P, either alone or in combination with traditional anti-cancer treatments, including the administration of chemotherapeutic agents, such as an anthracycline. To this end, a monoclonal antibody (mAb) has been developed that is specific for S1P, which can selectively adsorb S1P from the serum, acting as a molecular sponge to neutralize extracellular S1P. Since S1P has been shown to be pro-angiogenic, an added benefit to the antibody's effectiveness can be derived from the antibody's ability to starve the blood supply of the growing tumor. Thus, another sphingolipid-based anti-neoplastic strategy involves combining known activators of CER and SPH production (doxorubicin, doxorubicin, radiation therapy) coupled with a strategy to reduce S1P levels.

While sphingolipid-based anti-cancer strategies that target key enzymes of the sphingolipid metabolic pathway, such as SPHK, have been proposed, S1P itself has not been emphasized, largely because of difficulties in attacking this and related targets. As described herein, a highly specific monoclonal antibody to S1P has been produced that recognizes S1P in the physiological range and is capable of neutralizing S1P by molecular combination. Use of this antibody (and its derivatives) will deprive growing tumor cells of an important growth and survival factor. Moreover, use of such an antibody-based cancer therapy could also be effective when used in combination with conventional cancer treatments, such as surgery, radiation thereapy, and/or the administration of cytotoxic anti-cancer agents. An antibody-based combination therapy may improve the efficacy of chemotherapeutic agents by sensitizing cells to apoptosis while minimizing their toxic side effects, although administration of the antibody alone may also have efficacy in delaying the progression of disease. Indeed, the ability of the anti-S I P mAb to retard tumor progression in mouse models of human cancer and in allograft mouse models demonstrates the utility of anti-S1P antibody approaches in treating both human and animal tumors. Moreover, the discovery that several human cancers types (e.g., ovarian, breast, lung, and melanoma) can be treated in xenograft models demonstrates that the anti-S I P antibody approaches are not limited to one cancer cell or tissue type.

### C. Sphingolipids and Angiogenesis.

Angiogenesis is the process by which new blood vessels are formed from existing blood vessels. The angiogenesis associated with solid and circulating tumors is now considered to be a crucial component of tumorigenesis, as today the view that tumor growth is dependent upon neovascularization is scientifically well accepted.

S1P stimulates DNA synthesis and chemotactic motility of human venous endothelial cells (HUVECs), while inducing differentiation of multicellular structures essential early blood vessel formation. S1P also promotes the migration of bone marrow-derived endothelial cell precursors to neovascularization sites, and cells that over-express S1P receptors are resistant the anti-angiogenic agents, thalidomide and Neovastat. Thus, S1P, and particularly S1 receptors, are required for angiogenesis and neovascularization. Finally, cross-talk occurs between S1P and other pro-angiogenic growth factors such as VEGF, EGF, PDGF, bFGF, and IL-8. For example, S1P transactivates EGF and VEGF2 receptors, and VEGF up-regulates S1P receptor expression (Igarashi, Erwin et al. 2003).

As will be appreciated, clinical control of angiogenesis is a critical component for the treatment of cancer and other angiogenesis-dependent diseases such as age-related macular degeneration (AMD) and endometriosis. Anti-angiogenic therapeutics are also particularly attractive because the vascular endothelial cells that are involved in tumor angiogenesis do not mutate as easily as do cancer cells; consequently, vascular endothelial cells are less likely than cancer cells to gain resistance to prolonged therapy, making them useful therapeutic targets.

There are several lines of evidence suggesting that S1P is a potentially significant pro-angiogenic growth factor that may be important in tumor angiogenesis, including that: anti-S 1 P antibodies can neutralize S 1P-induced tube formation, migration of vascular endothelial cells, and protection from cell death in various *in vitro* assays using HUVECs; injection of breast adenocarcinoma MCF-7 cells expressing elevated S1P levels into mammary fat pads of nude mice results in an increase of angiogenesis-dependent tumors that are both larger and more numerous than when control cells are used; anti-S1P antibodies can dramatically reduce tumor-associated angiogenesis in an orthotopic murine melanoma allograft model; S1P increases new capillary growth into Matrigel plugs implanted in mice, an effect that can be neutralized by the systemic administration of anti-S 1 P antibodies; *in vivo* administration of anti-S1P antibodies can completely neutralize pro-angiogenic growth factor-induced angiogenesis (e.g., by bFGF and VEGF) in murine Matrigel plug assays; S1P stimulates the release of bFGF and VEGF from tumor cells *in vitro* and *in vivo,* an effect that can be reversed by anti-S1P antibodies; S1P enhances *in vitro* motility and invasion of a large number of different types of cancer cells, including glioblastoma multiforme cells; and anti-S I P antibodies significantly reduce the neovascularization associated with animal models of AMD.

The importance of S1P in the angiogenic-dependent tumors makes S1P an excellent target for cancer treatment. Indeed, antibody neutralization of extracellular S1P may result in a marked decrease in cancer progression in mammals, including humans, as a result of inhibition of blood vessel formation with concomitant loss of the nutrients and oxygen needed to support tumor growth. Thus, anti-S1P antibodies have several mechanisms of action, including: (1) direct effects on tumor cell growth; (2) indirect anti-angiogenic effects on vascular endothelial cells; and (3) the indirect anti-angiogenic effects that prevent the release and action of other pro-angiogenic growth factors. Accordingly, anti-S1P antibodies can also serve as anti-metastatic therapeutics, in addition to an anti-angiogenic therapeutics. They will also be useful in treating other hyperproliferative disorders associated with S1P activity, such as those cause by aberrant endothelial cell proliferation, as occurs with the angiogenesis associated with AMD.

### D. S1P Fibrogenesis and Scaring.

### i. S1P, fibroblasts and the remodeling process

It is clear that cardiac fibroblasts, particularly myofibroblasts, are key cellular elements in scar formation in response to the cell death and inflammation of a myocardial infarction (MI). Myofibroblast collagen gene expression is a hallmark of remodeling and necessary for scar formation. In addition to its other activities, S1P is also an inflammatory mediator that makes profound contributions to wound healing by activating fibroblast migration and proliferation, in addition to activating platelets, stimulating angiogenesis, and promoting smooth muscle function. Thus, S1P, perhaps produced locally by injured myocardium, could, in part, be responsible for the maladaptive wound healing associated with cardiac remodeling and failure, particularly by activating myofibroblasts in the heart.

There are three general responses of cells to S1P: protection from cell death; stimulation of proliferation; and the promotion of migratory responses. Accordingly, S1P activity or involvement with a particular disorder, cell line, etc. can be assessed by adapting assays of this sort for this purpose. There is evidence that fibroblasts respond to S1P in all three ways to promote wound healing. For instance, in several of the examples in the Example section below, evidence is presented that demonstrates that S1P contributes to remodeling by promoting cardiac myofibroblast activity (proliferation, migration, and collagen gene expression).

### ii. S1P and protection from cell death

As is the case for many cell types, fibroblasts are directly protected from apoptosis by addition of S1P, and apoptosis is enhanced by inhibitors of SPHK, and S1P blocks cytochrome C release and the resultant caspase activation. Further, fibroblasts transfected with SPHK1 exhibit protection from apoptosis, an effect that may depend upon translocation of SPHK1 to the plasma membrane. It is well-established that SPHK1 up-regulates Akt, thereby regulating Bcl-2 family members and protecting from apoptosis. Also, S1P₃ is required for Akt phosphorylation in mouse embryonic fibroblasts (MEFs). Also, up-regulation of SPHK and resulting increases in S1P levels protect cardiofibroblasts from apoptosis.

Ceramide, an upstream metabolite of S1P, decreases mitochondrial membrane potential coincident with increasing the transcription of death inducing mitochondrial proteins. Because of the rheostat mechanism, S1P may have the opposite effect and protect cardiac myofibroblasts (i.e., fully differentiated fibroblasts in the heart) from apoptosis. Indeed, S1P may even activate autophagy as a protection mechanism. These effects could be reversed by the neutralizing anti-S1P antibodies (or other molecules that bind and act to sequester S1P).

### iii. S1P induces fibroblast proliferation, differentiation, and promotes collagen gene expression

It has been demonstrated that fibroblasts respond to S1P treatment by increasing DNA synthesis, and fibroblasts transfected with SPHK1 exhibit increased cellular proliferation. Similar to its effects on non-cardiac fibroblasts, S1P is believed to stimulate cardiofibroblast proliferation (and subsequent differentiation). This effect occurs during remodeling and is another mechanism that explains S1P's maladaptive behavior (in this case, scar formation), particularly since S1P stimulates proliferation in multiple cell types, and results in S 1P-dependent DNA synthesis in cultured cardiofibroblasts (see Example 14, below).

A salient characteristic of fibroblasts, including cardiac myofibroblasts, is their ability to express collagen and lay down scar. It is well known that TGFβ up-regulates collagen production and promotes fibrosis in the remodeling heart. TGFβ has been shown specifically in cardiac fibroblasts to up-regulate several pro-fibrotic proteins, convert fibroblasts to myofibroblasts, and stimulate inflammatory protein expression. Interestingly, TGFβ increases SPHK mRNA, protein, and activity associated S1P levels, and up-regulation of TIMP1 by TGFβ is blocked by siRNA for SPHK and TIMP1. TIMP1 is generally expressed in cells transitioning from fibroblasts to myofibroblasts. Also, TGFβ-stimulated transition to myofibroblasts requires constitutive phosphorylation of FAK, which is regulated by signaling through S1P₁. Thus, signaling by TGFβ is closely linked to S1P. It has also been established that proliferating fibroblasts do not have high levels of collagen expression, while non-proliferating fibroblasts can be stimulated to transition to myofibroblasts and express large amounts of alpha smooth muscle actin (αSMA).

### iv. S1P induces migration in fibroblasts

Migration is necessary for cardiac fibroblast invasion of an infarcted area. S1P is likely involved in this process due to its profound stimulation of migration in other cell types, and thus may contribute to fibrosis. Reducing fibrosis would reduce scar formation and, in the context of cardiac tissue, would allow for improved heart function after a myocardial infarction (MI). Recognizing that some scar formation is necessary, however, to prevent cardiac rupture in the immediate post-MI period, it would be desirable to initiate limiting scar formation after the time that the risk of cardiac rupture subsides, particularly in the peri-infarct zone but also in the infarct zone itself.

It has also been demonstrated that S1P activates signaling systems, especially Rho, and resulting gene expression is consistent with its substantial effects on cellular migration. While it S1P₁ is required for mitogenicity and survival effects of fibroblasts, S1P₁ expression is associated with enhanced cell migration.

Assembly of contractile actin/myosin filaments is controlled by Rho/Rac/Cdc42 system and activation of all three Rho GTPases is necessary for cellular migration to take place. It is necessary for all three Rho GTPases to be expressed for migration to take place, but their localization of expression must vary for the coordination of their separate activities. For example, Rac and Cdc42 are responsible for lamellipodia and filopodial protrusion formation through actin polymerization. Importantly, Rho, Rac, and Cdc42 are responsible for S1P stimulated cellular migration. S1P₂, S1P₃, and S1P₄ activate Rho through coupling to G₁₃. The activation of these Rho GTPases by S1P is thus believed to be responsible for migration of cardiac fibroblasts in response to the wound created by an acute MI.

The examples in the Examples section below provide strong evidence that specific, sensitive anti-S 1P antibodies can act as molecular sponges to selectively absorb and neutralize S1P so that it cannot bind to the complement of S1P receptors on the surfaces of fibroblasts and inflammatory cells, thus decreasing inflammation and scaring. The effective extracellular concentration of S1P would thus be lowered by such a molecular sponge much in the same way anti-TNFα antibodies and receptor decoys (Embrel, Remicade) neutralize TNFα or the mAb sponge, Avastin, neutralizes the pro-angiogenic growth factor, vascular endothelial growth factor (VEGF).

### 2. Binding Sphingolipids for Therapeutic Benefit.

The methods and compositions disclosed herein, whether based on monotherapy or combination therapy, are said to be "sphingolipid-based" in order to indicate that these therapies can change the relative, absolute, or available concentration(s) of certain disease- or disorder-associated sphingolipids. Examples of disease- and disorder-associated sphingolipids, particularly hyperproliferative disorder-associated sphingolipids include, but are not limited, to ceramide (CER), sphingosine-1-phosphate (S1P), and sphingosine (SPH).

One way to control the amount hyperproliferative disorder-associated sphingolipids in a patient is by providing a composition that binds one or more sphingolipids or sphingolipid metabolites. Antibodies and other compounds that provide such binding may, for example, be used as therapeutic "sponges" that reduce the level of one or more free sphingolipid species in tissues and extracellular fluids, particularly blood. By "sponge" is meant that the sphingolipid-binding molecule (i.e., an anti-sphingolipid molecule), particularly an S1P-binding molecules (i.e., an anti-S1P molecule), specifically interacts with the target sphingolipid. Antibodies and other compounds that bind to cellular receptors of sphingolipids may also (or alternatively) be used to compete with and/or prevent sphingolipids from binding to receptors.

### A. Antibodies that Bind Sphingolipids

One aspect disclosed herein concerns antibodies that bind sphingolipids, particularly S1P, that can be delivered to a patient to provide treatment for a hyperproliferative disorder, particularly an S1P-associated hyperproliferative disorder. Such methods may, by way of nonlimiting example, (1) modulate the effective concentration of a specific sphingolipid or metabolite (e.g., S1P), (2) sterically inhibit the binding of a sphingolipid or a sphingolipid metabolite to a cellular receptor therefor, or to lower the concentration of a sphingolipid that is available for binding to such a receptor; (3) sterically inhibit the enzymatic conversion of a sphingolipid or a sphingolipid metabolite; or (4) remove sphingolipid or a sphingolipid metabolite from blood in vivo or ex vivo. In preferred embodiments, such antibodies are used as part of a combination therapy, while in other embodiments, they (or one or more of their antigen-binding domains) are incorporated into an agent that contains other moiety that binds to or otherwise specifically interacts with a different molecular species than that of the anti-sphingolipid moiety.

The term "antibody" is meant to encompass an immunoglobulin molecule obtained by in vitro or in vivo generation of an immunogenic response, and includes polyclonal, monospecific, and monoclonal antibodies, as well as T cell receptors, and fragments and derivatives thereof. An "immunogenic response" is one that results in the production of antibodies directed to one or more epitopes of an antigen. An "epitope" is a single antigenic determinant in a molecule.

Polyclonal antibodies are generated in an immunogenic response to an antigen (very often a protein or polypeptide) having many epitopes, and thus generally include a population of different antibodies directed to different epitopes within the antigen. Methods for producing polyclonal antibodies are well known in the art (see, e.g., Cooper et al, Section III of Chapter 11 in: Short Protocols in Molecular Biology, 2nd Ed., Ausubel et al., eds., John Wiley and Sons, New York, 1992, pages 11-37 to 11-41).

Monospecific antibodies are generated in a humoral response to a short (typically, 5 to 20 amino acids) immunogenic polypeptide that corresponds to a few (preferably one) isolated epitopes of the protein from which it is derived. A plurality of monospecific antibodies includes a variety of different antibodies directed to a specific portion of the protein, i.e., to an amino acid sequence that contains at least one, preferably only one, epitope. Methods for producing monospecific antibodies are known in the art (see, e.g., Id., pages 11-42 to 11-46).

A monoclonal antibody is a specific antibody that recognizes a single, specific epitope of an antigen. In a population of a monoclonal antibody molecules, each antibody molecule is identical to the others in the population. In order to isolate a monoclonal antibody, a clonal cell line that expresses, displays, and/or secretes a particular monoclonal antibody is first identified. This clonal cell line can be used to produce the desired monoclonal antibodies. Methods for the preparation of clonal cell lines and of monoclonal antibodies expressed thereby are known in the art (see, for example, Fuller et al, Section II of Chapter 11 in: Short Protocols in Molecular Biology, 2nd Ed., Ausubel et al., eds., John Wiley and Sons, New York, 1992, pages 11-22 to 11-11-36).

T cell receptors (TCR) are a distinct class of proteins that are genetically and structurally related to antibodies. TCR proteins belong to the immunoglobulin superfamily and have molecular structures similar to those of antibodies. Like antibodies, TCRs specifically recognize (i.e., specifically and bind) specific ligands. Complexes of TCRs are displayed on T cells and bind specific antigens for the purpose of triggering molecular events associated with T cell differentiation and activation. Like antibodies, TCRs recognize particular antigens. However, because of differences in the precise structures of the portions of TCR proteins that bind ligands and the amino acid sequences associated with those structures, as well as different mechanisms by which genes encoding a protein are diversified by rearrangement and mutation.

Antibody fragments and derivatives are proteins that are derived from antibodies and T-cell receptors and which retain the ability to specifically recognize the ligand recognized by the "parent" antibody or TCR. Preferred fragments include Fab fragments (i.e., an antibody fragment that contains the antigen-binding domain and comprises a light chain and part of a heavy chain bridged by a disulfide bond); Fab' (an antibody fragment containing a single anti-binding domain comprising an Fab and an additional portion of the heavy chain through the hinge region); F(ab')2 (two Fab' molecules joined by interchain disulfide bonds in the hinge regions of the heavy chains; the Fab' molecules may be directed toward the same or different epitopes); and a bispecific Fab (an Fab molecule having two antigen binding domains, each of which may be directed to a different epitope).

Single chain antibodies (scFv) comprise a variable, antigen-binding determinative region of a single light and heavy chain of an antibody linked together by a chain of 10-25 amino acids. U.S. patent numbers 5,260,203; 5,869,620; 5,455,030; 5,518,889; 5,534,621; 4,946,778; 6,025,165; and 6,027,725.

Complexes of single chain antibodies are also within the scope of the invention and include, but are not limited to, a disulfide-linked Fv, or dsFv (the variable, antigen-binding determinative region of a single light and heavy chain of an antibody linked together by a disulfide bond; a bispecific sFv (a scFv or a dsFv molecule having two antigen-binding domains, each of which may be directed to a different epitope); a diabody (a dimerized scFv formed when the VH domain of a first scFv assembles with the VL domain of a second scFv and the VL domain of the first scFv assembles with the VH domain of the second scFv; the two antigen-binding regions of the diabody may be directed towards the same or different epitopes); and a triabody (a trimerized sFv, formed in a manner similar to a diabody, but in which three antigen-binding domains are created in a single complex; the three antigen binding domains may be directed towards the same or different epitopes).

The term "antibody" also includes genetically engineered antibodies and/or antibodies produced by recombinant DNA techniques and "humanized" antibodies. Humanized antibodies have been modified, by genetic manipulation and/or in vitro treatment to be more human, in terms of amino acid sequence, glycosylation pattern, etc., in order to reduce the antigenicity of the antibody or antibody fragment in an animal to which the antibody is intended to be administered.

### B. A Preferred anti-S1P Monoclonal Antibody

A preferred biospecific monoclonal anti-S1P antibody (anti-S1P mAb) has been developed, and has been deposited with the A.T.C.C. and assigned accession number 306D326.1#26. This antibody can be used as a therapeutic molecular sponge to selectively absorb S1P and thereby thus lower the effective *in vivo* extracellular S1P concentrations for the purpose of treating hyperproliferative disorders that associated with S1P activity. This can result in the reduction of tumor volume and metastatic potential, as well as the simultaneous blockage of new blood vessel formation that otherwise can feed the growing tumor. This antibody (and molecules having an equivalent activity) can also be used to treat other hyperproliferative disorders impacted by S1P, including unwanted endothelial cell proliferation, as occurs, for example, in age-related macular degeneration and endometriosis, disorders related to fibrogenesis, and in many cancers. In addition, the ability of S1P to protect cells from apoptosis can be reversed by the agents such as the antibody, thus increasing the efficacy of standard pro-apoptotic chemotherapeutic drugs.

### 3. Pharmaceutical Compositions.

Another aspect of the present disclosure is drawn to compositions, including but not limited to pharmaceutical and/or biological compositions. According to the invention, a "composition" refers to a mixture comprising at least one carrier, preferably a physiologically acceptable carrier, and one or more therapeutic agents according to the invention. The term "carrier" defines a chemical compound that does not inhibit or prevent the incorporation of therapeutic agents into cells or tissues. A carrier typically is an inert substance that allows an active ingredient to be formulated or compounded into a suitable dosage form (e.g., a pill, a capsule, a gel, a film, a tablet, a microparticle (e.g., a microsphere), a solution etc.). A "physiologically acceptable carrier" is a carrier suitable for use under physiological conditions that does not abrogate (reduce, inhibit, or prevent) the biological activity and properties of the compound. For example, dimethyl sulfoxide (DMSO) is a carrier as it facilitates the uptake of many organic compounds into the cells or tissues of an organism. Preferably, the carrier is a physiologically acceptable carrier, preferably a pharmaceutically or veterinarily acceptable carrier, in which the therapeutic agent is disposed. A "pharmaceutical composition" refers to a composition wherein the carrier is a pharmaceutically acceptable carrier, while a "veterinary composition" is one wherein the carrier is a veterinarily acceptable carrier. The term "pharmaceutically acceptable carrier" or "veterinarily acceptable carrier" includes any medium or material that is not biologically or otherwise undesirable, i.e., the carrier may be administered to an organism along with a therapeutic agent, composition or compound without causing any undesirable biological effects or interacting in a deleterious manner with the complex or any of its components or the organism. Examples of pharmaceutically acceptable reagents are provided in The United States Pharmacopeia, The National Formulary, United States Pharmacopeial Convention, Inc., Rockville, Md. 1990.

The compositions disclosed herein can further comprise other chemical components, such as diluents and excipients. A "diluent" is a chemical compound diluted in a solvent, preferably an aqueous solvent, that facilitates dissolution of the therapeutic agent in the solvent, and it may also serve to stabilize the biologically active form of the therapeutic agent or one or more of its components. Salts dissolved in buffered solutions are utilized as diluents in the art. For example, preferred diluents are buffered solutions containing one or more different salts. A preferred buffered solution is phosphate buffered saline (particularly in conjunction with compositions intended for pharmaceutical administration), as it mimics the salt conditions of human blood. Since buffer salts can control the pH of a solution at low concentrations, a buffered diluent rarely modifies the biological activity of a therapeutic agent.

An "excipient" is any more or less inert substance that can be added to a composition in order to confer a suitable property, for example, a suitable consistency or to form a drug. Suitable excipients and carriers include, in particular, fillers such as sugars, including lactose, sucrose, mannitol, or sorbitol cellulose preparations such as, for example, maize starch, wheat starch, rice starch, agar, pectin, xanthan gum, guar gum, locust bean gum, hyaluronic acid, casein potato starch, gelatin, gum tragacanth, methyl cellulose, hydroxypropylmethyl-cellulose, polyacrylate, sodium carboxymethylcellulose, and/or polyvinylpyrrolidone (PVP). If desired, disintegrating agents can also be included, such as cross-linked polyvinyl pyrrolidone, agar, or alginic acid or a salt thereof such as sodium alginate. Other suitable excipients and carriers include hydrogels, gellable hydrocolloids, and chitosan.

The compositions can be formulated in any suitable manner. Therapeutic agents may be uniformly (homogeneously) or non-uniformly (heterogeneously) dispersed in the carrier. Suitable formulations include dry and liquid formulations. Dry formulations include freeze dried and lyophilized powders, which are particularly well suited for aerosol delivery to the sinuses or lung, or for long term storage followed by reconstitution in a suitable diluent prior to administration. Other preferred dry formulations include those wherein a composition according to the invention is compressed into tablet or pill form suitable for oral administration or compounded into a sustained release formulation. When the composition is intended for oral administration but the therapeutic agent is to be delivered to epithelium in the intestines, it is preferred that the formulation be encapsulated with an enteric coating to protect the formulation and prevent premature release of the therapeutic agents included therein. As those in the art will appreciate, the compositions of the invention can be placed into any suitable dosage form. Pills and tablets represent some of such dosage forms. The compositions can also be encapsulated into any suitable capsule or other coating material, for example, by compression, dipping, pan coating, spray drying, etc. Suitable capsules include those made from gelatin and starch. In turn, such capsules can be coated with one or more additional materials, for example, and enteric coating, if desired. Liquid formulations include aqueous formulations, gels, and emulsions.

Liquid pharmaceutical compositions which are sterile solutions or suspensions can be utilized by for example, intramuscular, intrathecal, epidural, intraperitoneal or subcutaneous injection. Sterile solutions can also be administered intravenously. The active ingredient may be prepared as a sterile solid composition that may be dissolved or suspended at the time of administration using sterile water, saline, or other appropriate sterile injectable medium. Carriers are intended to include necessary and inert binders, suspending agents, lubricants, flavorants, sweeteners, preservatives, dyes, and coatings.

Those skilled in the art will appreciate that when the compositions disclosed herein are administered as agents to achieve a particular desired biological result, which may include a therapeutic or protective effect(s) (including vaccination), it may be necessary to combine the therapeutic agents with a suitable pharmaceutical carrier. The choice of pharmaceutical carrier and the preparation of the therapeutic agent as a therapeutic or protective agent will depend on the intended use and mode of administration. Suitable formulations and methods of administration of therapeutic agents include those for oral, pulmonary, nasal, buccal, occular, dermal, rectal, or vaginal delivery.

Pharmaceutical compositions of the present invention can be used in the form of a solid, a solution, an emulsion, a dispersion, a micelle, a liposome, and the like, wherein the resulting composition contains one or more of the compounds, as an active ingredient, in admixture with an organic or inorganic carrier or excipient suitable for enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers that can be used include glucose, lactose, mannose, gum acacia, gelatin, mannitol, starch paste, magnesium trisilicate, talc, corn starch, keratin, colloidal silica, potato starch, urea, medium chain length triglycerides, dextrans, and other carriers suitable for use in manufacturing preparations, in solid, semisolid, or liquid form. In addition auxiliary, stabilizing, thickening and coloring agents and perfumes may be used.

A therapeutic kit as disclosed herein comprises a reagent as disclosed herein with one or more additional components, including vials or other containers for storage of a composition according to the invention, instructions for use, and packaging materials.

### EXAMPLES

The following Examples are provided to illustrate certain aspects of the present invention and to aid those of skill in the art in practicing the invention. These Examples are in no way to be considered to limit the scope of the invention in any manner.

The examples in this Example section demonstrate favorable outcomes from pharmacokinetic and toxicology studies in animal models of human and animal tumors. The S1P-responsiveness of multiple tumor cell lines, including, but not limited to HeLa cells (human cervical adenocarcinoma), U-87 (human brain glioblastoma), U266 (human multiple myeloma), A549 (human lung carcinoma), U937 (human histocytic lymphoma), MCF-7 (human mammary gland adenocarcinoma), SKOV3 (human ovarian cancer), OVCAR3 (human ovarian cancer), MDA MB 231 (human breast cancer), MDA MB 468 (human breast cancer), H929 (human myeloma), RPMI-8226 (human multiple myeloma, U937 (human lymphoma), SKBR-3 (human breast cancer), and HT-29 (human colorectal adenocarcinoma) cells, is also described. These tumor cell lines represent a spectrum of histological subtypes, genetic aberration and levels of the receptors and enzymes producing and metabolizing S1P. This includes cellular proliferation, motility, invasion, apoptosis, and, for a select group, production of angiogenic factors. It is also demonstrated that many tumor cell lines are also S1P-responsive in their abilities to escape apoptosis induced by the representative chemotherapeutic agents doxorubicin and paclitaxel.

The S1P-induced protection from apoptosis can also be reversed in the presence of an anti-S1P agent, the anti-S1P mAb. An important characteristic of metastatic cancers is that the tumor cells escape contact inhibition and can migrate away from their tissue of origin. The potent ability of S1P to induce cell invasion in multiple tumor cell lines is also reported, as is the ability of the anti-S1P mAb to inhibit the metastatic potential of S1P. In a limited number of cell types, S1P promotes cell proliferation above the already substantial basal levels. Importantly, *in vivo* xenograft studies demonstrate that the anti-S1P mAb reduces tumor volume in mice given a variety of human cancer cells and one mouse melanoma cell line (B16-F10).

S1P has been shown to promote angiogenesis by the migration of Human Umbilical Vein Endothelial Cells (HUVECs) both *in vitro* and *in vivo.* The studies described below confirm that S1P stimulates the formation of capillary-like tubules *in vitro* and *in vivo.* Moreover, this process can be inhibited by the anti-S1P mAb. For example, *in vivo* Matrigel plug assays reveals that the anti-S1P mAb is anti-angiogenic. This was confirmed *in vitro* using HUVECs. Thus, it has been shown that S1P not only protects HUVECs from doxorubicin and paclitaxel-induced apoptosis, but it also stimulates HUVEC migration and blood vessel formation. Further, examples are presented demonstrating the ability of the anti-S1P mAb to reduce tumor angiogenesis in vivo using a B16-F10 allograft model. All these effects are mitigated by the anti-S1P mAb.

In addition to S1P produced by tumor cells themselves, serum is a rich source of this important tumorigenic factor. Anti-S1P agents such as an anti-S1P mAb can neutralize S1P present not only in the serum but also in the vicinity of solid tumors.

Examples are presented to illustrate the pleiotropic effects of S1P as a tumorigenic growth factor in several tumor-derived human cell lines, suggesting that our anti-S1P mAb may be successfully used in a variety of cancer types. Further, data presented demonstrates that mouse-derived tumors can be treated with the anti-S1P mAb, suggesting a veterinary application of the antibody.

### Example 1

### Anti-S1P mAb alone decreases tumor progression

The anti-tumor efficacy of an anti-S1P monoclonal antibody (mAb) was evaluated in two orthotopic breast cancer models and one ovarian cancer model. Tumors were developed by injection of MDA MB 231 human tumor cells into the mammary fat pads of nude (NCr Nu/Nu) mice using standard protocols. After 10 days, when solid tumors had formed (∼100mm³), intraperitoneal treatments of anti-S1P mAb or vehicle alone were initiated. The anti-S1P mAb was administered 25 mg/kg intraperitoneally (i.p.) every other day in saline. Treatments were administered every other day for the duration of the study. Tumor volumes were also determined and recorded every other day. The study was concluded and the animals were sacrificed when the tumors reached their maximal size as defined by IACUC standards (about 1.5 cm³). Tumors were harvested, measured, and processed for immunohistochemical evaluations of micro-vascular changes.

The efficacy of the anti-S1P mAb in reducing tumor volume over time has been demonstrated. The ability of the anti-S1P mAb to reduce tumor volume was apparent only after the tumors reached approximately 400mm³. At this point, the tumors from the control animals continued to grow, while the tumors from the anti-S1P treated animals nearly stopped growing. At the end of the study, tumor volumes were reduced by 60% (p<0.001 by ANOVA) in the antibody treated animals. The anti-S1P mAb significantly reduced the final tumor weights by an average of 40% when compared to tumors from control treated animals.

Favorable *in vivo* efficacy data has also been obtained with MDA MB 468 human breast cancer cells in an equivalent orthotopic mammary fat pad model. In this model, a 40% reduction in average tumor volume was observed for the antibody-treated animals. The reduction in the size of tumors from animals treated with the anti-S1P mAb correlated to reduced serum levels of the pro-angiogenic and tumorigenic factor, IL-8. Thus, in both of the xenograft models tested, the anti-S1P antibody markedly inhibited tumor growth.

### Example 2

### Anti-S1P mAb inhibits tumor angiogenesis in vivo

To investigate the ability of anti-S1P mAb to neutralize the pro-angiogenic effects of S1P, an *in vivo* Matrigel Plug assay was used. This assay is a well-established animal model for tumor angiogenesis using Matrigel, a proprietary mixture of tumor remnants including basement membranes derived from mouse tumors. When Matrigel is injected subcutaneously (s.c.) into an animal, it forms a `plug'. Upon addition of angiogenic factors, the plug is invaded by vascular endothelial cells, which then form capillary-like blood vessels. Matrigel can be prepared either alone or mixed with recombinant growth factors (rGF) such as FGF or VEGF as a pro-angiogenic compounds, then injected s.c. in the back of 6 week old C57B1/6N female mice. Endogenous S1P from the blood and surrounding tissue could supply the plug with an additional pro-angiogenic stimulus. Based on the *in vivo* performance characteristics of the antibody (see below), it was presumed that treatment of mice with the anti-S1P mAb would reduce available serum and tissue S1P levels and, consequently, reduce the concentration of endogenous S1P available to the plug. In these experiments, the ability of the antibody to reduce angiogenesis in an optimally stimulated plug (added protein growth factors, plus endogenous S1P) was studied. One group of mice that received Matrigel containing hGF also received intraperitoneal (i.p.) injections of anti-S1P mAb every 48 hr. starting 1 day prior to Matrigel implantation. Each treatment group (Matrigel, Matrigel plus hGF, or Matrigel plus hGF with mAb treatment) consisted of a minimum of six mice. After 10 days, the mice were heparinized and injected with the fluorescent lectin, Isolectin B4-FITC, which binds to adhesion molecules expressed by vascular endothelial cells. The plugs were then excised. Visual examination of the plugs revealed that the control (Matrigel only) plugs were colorless, whereas those plugs containing hGF had clearly undergone angiogenesis as indicated by the red, bloody appearance. The plugs from animals treated with the anti-S 1P mAb and containing hGF were colorless, thus suggesting an inhibition of micro-vascularization. The plugs were then embedded in OCT freezing medium and sectioned. Micro-vascular density was qualitatively accessed by lectin-FITC stained vessels, as shown in Figure 5. Blood vessel staining was sporadic in control (untreated) plugs, whereas the plugs containing hFGF demonstrated significant evidence of vascularization (middle photo of panel C). The plugs from mice treated with the anti-S1P mAb demonstrated a significant reduction in blood vessel formation compared to the hGF plugs from untreated mice (no mAb). Quantification of stained vessels revealed an 11-fold decrease in neo-vascularization of hGF containing plugs from animals treated with the antibody in comparison to non-treated animals. This evaluation further demonstrates the ability of endogenous serum and tissue S1P to enhance micro-vascularization as well as the ability of the anti-S1P mAb to neutralize endogenous S1P's pro-angiogenic effects *in vivo.*

These results demonstrate the anti-angiogenic effects of the anti-S1P mAb *in vivo* and the dramatic effects of the anti-S1P mAb in reducing tumor progression without the benefit of cytotoxic chemotherapeutic agents. While not wishing to be bound to a particular theory, this data reveals that the anti-tumorigenic effects of the anti-S1P mAb may be due to the mitigation of the angiogenic effects of S1P that would normally promote tumor progression. Thus, some effective cancer treatments will result from the additive effects of the anti-S1P agent in combination with one or more other cytotoxic agents. *In vitro* and *in vivo* work demonstrating the additive anti-tumor effects of a combination treatment are described below.

### Example 3

### In vivo Pharmacokinetics and Toxicology

Prior to initiating *in vivo* studies, the toxological and pharmacokinetic characteristics of the anti-S1P mAb were determined in mice. The half-life of the antibody was measured to determine how to optimally dose the animals to maintain a reasonable blood level of the anti-S1P. mAb. Mice were dosed with 25mg/kg of the anti-S1P mAb intravenously (i.v.) and bled at designated time points. A competitive ELISA employing a Biotin-labeled anti-S 1P mAb was used to determine the concentration of antibody remaining in the mouse blood between 20 min. and 120 hr. after the bolus dose of antibody. Figure 6 demonstrates that the serum half-life of the mAb was approximately 20-25 hr. In addition to i.v. injections, mice were administered a bolus dose of anti-S 1 P mAb by intraperitoneal (i.p.) injection. After 20 minutes, over 95% of the antibody appeared in the bloodstream. Taken together, these data indicate that mice can effectively be dosed either i.p. or i.v. with the anti-S1P mAb.

Due to the pleiotropic nature of S1P, potential adverse effects on physiological functions that might be caused by a reduction of systemic S1P as a result of treatment with the anti-S I P mAb were investigated. Mice were treated with 1, 3, 10, 30, or 50mg/kg of the anti-S1P mAb or vehicle (PBS) for seven consecutive days by tail vein injection. Due to the long half-life of the antibody, simulations of the dosing regimen indicated that the animals accumulated over twice the amount of antibody over the 7 days. Twenty-four hours after the final treatment, the mice were sacrificed, biological fluid was collected, and organs were harvested. Even at the highest dose, all chemical and CBC panel analyses were within normal ranges. Furthermore, histopathological examination by a board certified veterinary pathologist revealed no lesions or other pathological changes in the liver, kidney, heart, lungs, or spleen of mice in any treatment group. Throughout the duration of the study, mice in all treatment groups consumed similar amounts of food and water and socialized no differently than control animals. Body weights and activity levels were also normal. Therefore, at all doses tested, including 50mg/kg, the antibody appeared to be well-tolerated.

The information from the pilot pharmacokinetic and toxicity studies provided insight as to how to dose animals in animal efficacy studies. A simulation of dosing of 10mg/kg anti-S I P mAb every third day demonstrates the constant presence of the antibody in the mouse that does not accumulate appreciably over time.

### Example 4

### Antibody Characteristics

One important performance characteristic of an antibody is its specificity towards its antigen, i.e., does it specifically react with its intended target. In a competitive ELISA using the anti-S1P mAb tested against a gold-standard S1P sample (obtained from Avanti Polar Lipids and confirmed by HPLC and mass spectroscopy) as well as several other lysolipid controls, the antibody demonstrated no cross-reactivity to sphingosine (SPH), the immediate metabolic precursor of S1P. Moreover, the anti-S1P mAb did not recognize lysophosphatic acid (LPA) or sphingosylphosphorylcholine (SPC). Both LPA and SPC are structurally similar to S1P.

Another important performance characteristic of a good therapeutic antibody is that it can recognize the target (i.e., S1P) in the physiological range. Studies using the industry standard HPLC technique for measuring serum S1P revealed that normal serum S1P levels are within the400-700 pmol/mL range, while patients with significant coronary artery disease display higher serum S1P levels, in the 900-2,500 pmol/mL range. Data indicates that ascites from ovarian patients contain a large amount of S1P that is close to serum S1P levels. The dynamic range of the anti-S1P mAb used in these examples, has been demonstrated and it has been indicated that the antibody is capable of recognizing S1P at both normal and clinically relevant S1P concentrations. Consequently, the anti-S1P mAb has a dynamic range that is sensitive as well as specific, i.e., it is "specifically reactive" with its intended target. A comparison between the industry standard HPLC measurement of human serum S1P from a normal volunteer and ELISA-based measurements using the anti-S I P mAb showed good correspondence between the two methods, thus validating the use of an ELISA as an accurate platform for S1P determination.

An additional important characteristic of an antibody is its ability to recognize its ligand in an *in vivo* environment. Accordingly, the ability of the anti-S1P mAb to recognize and selectively absorb S1P from human and mouse serum was studied in an *in vitro* assay utilizing both radioactivity and mass spectrometry analysis. The mAb was efficiently able to absorb up to 88% and 77% of ³H-S1P added to PBS and mouse serum, respectively. The difference in the ability of the mAb to absorb similar levels of the ³H-S1P in the mouse serum when compared to the control (PBS) was most likely due to the mAb also binding to endogenous S1P, which is present in large concentrations in mouse serum. These data are consistent with *in vitro* cellular bioassays performed in serum plus *in vivo* efficacy experiments, demonstrating that the mAb can effectively neutralize S1P in serum.

Taken together, these results demonstrate the successful development of biospecific monoclonal antibody to S1P that is both specific and sensitive. Thus, the mAb, and other agents capable of specifically reacting with S1P, can be used therapeutically as a molecular "sponge" or "sink" to efficiently and selectively absorb S1P from serum, thereby reducing its effective concentration in extracellular fluids *in vivo.* In addition, the anti-S1P mAb (and like reagents) can be used as a detection reagent in an assay to detect (quantitatively, semi-quantitatively, or qualitatively) levels of S1P (or other target analytes) in biological samples (e.g., tissue samples (e.g., from biopsies) or bodily fluids (e.g., blood, ascites, serum, lymph, saliva, urine, cerebrospinal fluid, etc.). Evaluation of S1P as a biomarker could be used in conjunction with genomic profiling of tissue S1P receptor levels and levels of SPHK to stratify patients by there dependence on S1P for tumor growth. Such assays will have application in "theranostic" platforms in which a patient's serum, ascites, or tumor biopsy material would be measured for S1P content, preferably paired with genomics analysis, thereby allowing it to be predicted which patients would most benefit from a therapeutic treatment that employs the detection reagent formulated as a therapeutic in a subsequently delivered therapy.

### Example 5

### Anti-S1P mAb Increases Chemotherapeutic-induced Tumor Cell Death

In addition to the pro-angiogenic properties of S1P (see above), it has been demonstrated that the actions of S1P in promoting tumor growth can be attributed to the molecule's ability to directly promote cell proliferation and to protect the cells against pro-apoptotic chemotherapeutic agents. The ability of S1P to block the up-regulation and activation of the apoptotic terminal effector, caspase-3, has been studied in several tumor cell lines when exposed to clinically-relevant levels of the chemotherapeutic agents, paclitaxel (*Taxol*) and doxorubicin (*Andriamycin*). The ability of S 1P to protect A549, HT-29, U266BL, and HeLa cells from apoptosis triggered by these chemotherapeutic agents has been demonstrated. In addition, it has been shown that paclitaxel and doxorubicin potently induced caspase-3 activation by 50-1000% after 48 hr. of treatment in media containing 10% serum. In an attempt to promote conditions resembling physiological levels of S1P, the 10% serum was supplemented with additional S1P (100nM), and then the cells were treated with cytotoxic agents. In comparison to cells treated with 10% serum, cells supplied with additional exogenous S1P were protected from paclitaxel- and doxorubicin-induced apoptosis. This was demonstrated by the significant (p<0.001) reduction in caspase activity seen in the presence of the added sphingolipid. Importantly, the mAb was effective in mitigating the protective effects of S1P in the presence of the chemotherapeutic agents. Even in the absence of added S1P, paclitaxel- and doxorubicin-induced caspase activation was enhanced by the anti-S1P mAb (25% and 50-200% increases, respectively), indicating that the protective anti-apoptotic effect of endogenous S1P was eliminated by selective antibody absorption of S1P present in the serum. Considering that serum has substantial endogenous S1P, the efficacy of the antibody in the absence of added S1P (third set of bars) shows that endogenous levels of S1P in the serum were sufficient to afford some protection against doxorubicin or paclitaxel-induced cell death.

The specificity of the anti-S1P mAb was demonstrated in control experiments utilizing a structurally similar bioactive lipid mediator and a nonspecific isotype matched monoclonal antibody. Experiments utilizing the A549, HT-29 and U266BL cell lines, LPA failed to reduce caspase activation. Moreover, the nonspecific monoclonal antibody failed to neutralize S1P-responsiveness, showing specificity of the anti-S1P mAb in mitigating S1P effects.

Similar data demonstrated the anti-apoptotic effects of S1P in U266BL, MCF-7, and HT-29 cells. However, not all tumor cell lines respond to S1P. For example, mouse melanoma B 16-F10, human lymphoma U937, and human ovarian MDA MB 2774 carcinoma cells did not respond to S1P when evaluated for the ability of the lipid mediator to protect those cell types from doxorubicin- or paclitaxel-induced cell death. Moreover, the anti-S I P mAb did not increase the killing potential of the chemotherapeutic agents, thus demonstrating the lack of effect that S1P exerts on these tumor cell lines.

### Example 6

### Anti-S1P mAb Inhibits Release of Tumor-Promoting Cytokines and VEGF

In animal models, expression of interleukin-6 and 8 are associated with increased tumorigenicity, ascites formation, angiogenesis, and invasiveness of ovarian cancer cells. In ovarian cancer patients, serum levels of IL-6 are elevated by several magnitudes. Taken together, these studies indicate that IL-6 is an important modulator or, at least, an indicator of ovarian cancer progression. For these reasons, it was decided to investigate whether an anti-S1P monoclonal antibody could reduce IL-6 production as a measure of the antibody's ability to reduce ovarian cancer progression. For these studies, it was demonstrated that 10µM S1P could stimulate IL-6 release from ovarian cancer cells. Culture supernatants of ovarian cancer OVCAR3 cells, treated with or without S1P, were collected and analyzed for IL-6 release into the cell-conditioned media using an ELISA. As demonstrated S1P increased the expression of IL-6 by an average of 275% when compared to non-treated cells. For cells pretreated with the anti-S1P mAb, IL-6 expression was significantly reduced. Increasing amounts of the mAb (from 0.01-10 µg/mL), resulted in a dose-dependent loss of IL-6 expression. Similar significant results were obtained utilizing two other neo-vascularization factors, IL-8 and VEGF, using several tumor cell lineages. These data show that the blockade of growth factor release is an additional effect of anti-S1P agents.

### Example 7

### Anti-S1P mAb Decreases S1P-Stimulated Increases in Cancer Cell Proliferation

The ability of S1P to increase proliferation of selected human-derived tumor cell lines including A549, HT-29, MCF-7 and HeLa cells by ³H-thymidine incorporation studies has been demonstrated. DNA synthesis was significantly (p<0.05) increased in cells treated with 10nM S1P when compared to non-treated control cells in each of these cancer cell lines. Even though tumor-derived cells normally have high basal levels of proliferation, S1P appears to augment proliferation in most tumor cell lines. Importantly, the increase in DNA synthesis stimulated by S1P was mitigated by the addition of 1µg/ml of the anti-S1P mAb. Similar data were obtained with the OVCAR3, MDA MB 273, and MDA MB 468, tumor cell lines using crystal violet staining.

### Example 8

### Anti-S1P mAb Decreases S1P-Stimulated Increases Tumor Cell Metastatic Potential

An important characteristic of metastatic cancers is that the tumor cells acquire the ability to migrate and invade tissues. S1P has been shown to promote metastatic potential in breast cancer, glioblastoma, and melanoma cells using *in vitro* cell invasion assays. It was decided to evaluate whether the anti-S1P monoclonal antibody could block S1P-mediated cell migration. To evaluate the chemotactic effects of S1P on tumor cells, an *in vitro* Matrigel cell invasion assay commonly used in chemoinvasion studies was used. As shown, treatment with levels of S I P found in human serum induced an increase in A549, HT-29 and MCF-7 cell invasion through the Matrigel matrix. A 6 to 9-fold increase in cell migration was obtained with 1 µM S1P when compared to non-treated control cells. Addition of the monoclonal anti-S1P antibody reduced tumor cell invasion to control levels. Four control experiments demonstrated the specificity of these effects. First, incubating A549 cells with LPA had no effect upon cell migration, demonstrating S1P's specific effect on this cell line. Second, addition of non-specific mouse IgG did not inhibit S1P-induced cell migration. Third, titrating down the concentration of anti-S1P mAb from 1 µg/mL to 0.001 µg/mL reduced the ability of the antibody to effectively neutralize all of the S1P. Fourth, B16-F10 cells (previously determined to be unresponsive to S1P; see Example 5) did not migrate upon incubation with S1P.

### Example 9

### In vitro Demonstration that Anti-S1P mAb Blocks Tumor Angiogenesis

The process of neo-vascularization is vital to the survival and growth of a tumor. Neo-vascularization is dependent upon the invasion, vessel formation, and survival of endothelial cells inside or adjacent to the growing tumor. This series of experiments describes the evaluation of the tumor-promoting ability of S1P to stimulate neo-vascularization in terms of tube formation, migration, and survival against chemotherapeutic agents.

S1P has been shown to promote the migration of Human Umbilical Vein Endothelial Cells (HUVECs) and the formation of *de novo* blood vessel formation *in vitro* using Matrigel and other similar assays. HUVECs isolated from human umbilical cords form tubular capillary-like structures when provided with critical growth factors. While antibodies directed against key protein growth factors like VEGF and FGF neutralize blood vessel formation and tumor growth, the anti-angiogenic effects of neutralizing antibodies directed against sphingolipid growth factors have not been examined previously. It has been demonstrated that HUVECs seeded onto growth factor-reduced Matrigel formed multiple capillary-like structures in the presence of physiologically relevant serum/plasma concentrations of S1P (400-700 pmol/mL). The HUVECs failed to form capillary-like structures in the absence of S1P. Moreover, a monoclonal antibody directed against S1P substantially reduced the formation of the typical capillary-like structures.

The ability of endothelial cell to migrate to the site of a tumor is also an important process during angiogenesis. The ability of physiological concentrations of S1P to stimulate HUVEC migration in the Matrigel chemoinvasion assay described above was determined. The potent ability of 0.1-1 µM SIP to stimulate HUVEC migration 2-2.5 fold over non-treated HUVECs has been demonstrated. Importantly, this stimulation of migration was completely neutralized by the addition of the anti-S1P monoclonal antibody.

The ability of endothelial cells to undergo angiogenesis and feed a growing tumor is also dependent upon the cells' ability to circumvent cell death induced by chemotherapeutic agents. The ability of S1P to potently protect HUVECs from cell death as assayed by caspase-3 activation has been demonstrated. The ability of S I P to protect cells from death was reversed by incubation with the anti-S1P mAb. Furthermore, similar to the assays described above, the anti-S1P mAb enhanced caspase-3 activation induced by doxorubicin and paclitaxel. These experiments were performed in the presence of 20% serum, demonstrating the ability of endogenous S1P in serum to protect HUVECs from cell death induced by chemotherapeutic agents.

These studies confirm that S1P is a potent pro-angiogenic growth factor that can influence *de novo* blood vessel growth and protect vascular endothelial cells from cytotoxic agents. These results demonstrate that an anti-S I P agent can exert an anti-angiogenic effect by several mechanisms, including one that enhances chemotherapy-induced cell death of endothelial cells. Moreover, such agents, in combination with standard chemotherapeutic agents, can act to reduce angiogenesis and slow cancer progression in the clinic. In sum, these results demonstrate that S1P is a pleiotropic tumorigenic growth factor that has profound effects on tumor cell proliferation, invasion (i.e., metastatic potential), neo-vascularization, and protection from apoptosis. In addition, S1P protects most cells types against apoptotic chemotherapeutics. Even though cell proliferation was significantly stimulated by S1P, the effects of S1P on the other parameters were much more dramatic and uniformly applicable to most of the cell types studied. The pro-angiogenic effects of S1P were dramatic, and anti-S1P agents such as the anti-S I P monoclonal antibody described herein block all of these effects. Additionally, the results demonstrate that agents such as the anti-S1P monoclonal antibody can block the production of other pro-angiogenic growth factors, providing an additional therapeutic mechanism for our anti-S1P mAb in halting tumor progression.

The efficacy of an anti-S1P agent to block the micro-vascularization of tumors as well as inhibiting tumor cell growth (volume and weight) has been demonstrated. Compelling data from screens of several cell lines derived from a variety of solid and circulating tumor types shows that anti-S1P agents (e.g., antibodies) can be useful in the treatment of many cancer types, particularly those that have a dependence on angiogenesis. The favorable *in vivo* pharmacokinetic and toxicology profiles of the agents such as the anti-S1P monoclonal antibody described herein further demonstrate that anti-S1P agents are likely to be drugable in humans.

### Example 10

### Anti-S1P mAb Blocks Tumor Angiogenesis in an in vivo Allograft Model

Growing tumors depend upon blood vessel growth. Agents that can inhibit this process without significant toxicity could serve as potent new anti-tumor therapeutics. Although the anti-VEGF antibody therapeutic, Avastin, was recently approved for clinical use for colon cancer therapy, Avastin has not proven effective in lung and breast cancer clinical trials. Therefore, additional approaches to inhibit tumor angiogenesis are still needed. As shown in Example 9, one such approach is to block the pro-angiogenic effects of S1P. The anti-S1P mAb has been shown to potently inhibit S1P-induced endothelial cell migration, capillary growth, and cell survival *in vitro.* The anti-S1P mAb has also been shown to neutralize S1P's ability to enhance *de novo* blood vessel formation in the *in vivo* murine Matrigel plug model of angiogenesis. Accordingly, the efficacy of anti-S1P to reduce the micro-vascularization of tumors in two *in vivo* murine models was investigated. As S1P has been shown to promote or enhance angiogenesis, the anti-S1P mAb was expected to suppress *de novo* blood vessel formation, and hinder tumor growth.

Based upon the *in vitro* studies described in Examples 5 and 8, it was known that the murine melanoma tumor-derived cell line B16-F10 was unresponsive to the direct effects of S1P. S1P did not induce proliferation, invasion, or protection from cell death in these cells, as it does in most other tumor cells. Thus, it was hypothesized that any anti-tumor effect of the anti-S1P mAb on B16-F10 tumors would arise not from inhibition of S1P-induced tumor growth, but from an inhibition of S1P-enhanced tumor-associated angiogenesis. An inhibition of neo-vascularization in the growing tumor would, thus, significantly slow tumor progression. Therefore, a study was undertaken to investigate the ability of the anti-S1P mAb to retard melanoma tumor growth after an orthotopic xenograft placement of the B16-F10 cells in mice.

In this model, tumors were developed in 4 week old female C57B1/J6 mice (the strain from which the melanoma cells were originally isolated) by implantation of B16-F10 cells into the right flanks of the mice. Tumors were allowed to establish to a volume of 100mm³, as determined by caliper measurements. When the tumors began reaching the desired volumes, mice were computer-randomized into treatment groups (n= 6-8). Mice with tumors between 75-150mm³ were selected for treatment. All animals containing tumors out of this volume range were not included in this study. The selected mice were then injected i.p. every three days with either the anti-S I P mAb (25mg/kg), an isotype matched non-specific mAb (25mg/kg; directed against a plant pathogen), or saline. All treatments were double-blinded. Tumor volumes were measured independently every day by two people and averaged. When tumor volumes began reaching the maximal size (about 1.8 cm³ by IACUC standards), all animals were sacrificed. Final tumor volumes and weights were recorded. Only after all data were analyzed was the study un-blinded.

A 60% reduction of tumor volume over time from mice treated with the anti-S 1P mAb in comparison to those animals treated with saline or the non-specific mAb has been demonstrated. The inhibition of tumor progression occurred by the reduction of neovascularization of the tumor has been confirmed. The reduction of tumor progression is believed to be directly related to the anti-angiogenic effects of the anti-S1P mAb. Further, these mice were not immune-compromised, indicating that blocking sphingolipid action can reduce tumor progression in normal animals. In addition, this study demonstrates that mouse-derived tumors can be treated with an anti-S1P antibody, indicating that the antibody will also be useful for veterinary applications aimed at cancer treatment, particularly in companion animals and livestock.

### Example 11

### Anti-S1P mAb in Combination with Chemotherapeutic Agents Decreases Tumor Progression

While Example 1 demonstrates that an anti-S 1P mAb is efficacious in reducing tumor size when administered alone, the treatment for human cancers may be more successful, or be applied to treat more types of cancer, if an agent that binds to and reduces the effective *in vivo* concentration of S1P is given in combination with one or more chemotherapeutic agents or as an adjunct to procedures such as surgery and radiation therapy. Indeed, when mice having fairly large tumors (for example, 700-800 mm³; established by implanting MDA MB 231 mammary carcinoma cells) were treated with the anti-S1P mAb (25 mg/kg every other day) either alone or in combination with one dose of Taxol (paclitaxel) at a bolus dose of 20 mg/kg, the combination demonstrated a synergistic effect in that the antibody-treated mice showed almost no further growth. Moreover, addition of the S1P binding agent to the chemotherapeutic treatment dramatically improved survivability of the mice.

### Example 12

### Anti-S1P mAb Administered Alone Eliminates Established Human Ovarian Tumors

While Examples 1 and 12 demonstrate that an anti-S1P mAb is efficacious in reducing tumor size when administered alone or in combination with cytotoxic agents, this example demonstrates that, using the right human tumor type, one can demonstrate elimination of established tumors, i.e., a cure may be effected.

It has been demonstrated that the anti-S1P mAb was efficacious in eliminating established orthotopic SKOV3 human ovarian tumors in nude mice. In this model, tumors were allowed to establish for two weeks prior to the initiation of treatment. MRI analysis revealed that all saline control mice contained large tumors throughout the peritoneal cavity and that these mice had accumulated observable amounts of ascites fluid. Conversely, in three out of the five animals treated with the anti-S1P mAb at 25mg/kg i.p. every three days, no tumors or ascites were detected during MRI analysis or upon dissection of the peritoneal cavity after termination. Only two out of the five animals treated with the anti-S1P mAb had detectable tumors; significantly, these tumors were 68% smaller (750mg versus 2300mg) than tumors from the saline-treated animals (*p<0.05). In addition, the animals treated with the anti-S1P mAb and no tumors had a large amount of subcutaneous fat around their bellies, confirming the normal body weights and over-all health exhibited by antibody-treated animals.

### Example 13

### Angiogenesis and Age-Related Macular Degeneration

The purpose of the experiments described in this example was to determine if an anti-S1P mAb could reduce the angiogenesis in a model other than tumor angiogenesis. For these studies, an established animal model of Age-related Macular Degeneration (AMD) was employed, namely choroidal neovascularization (CNV) by rupture of Bruch's membrane with laser burns using a slit lamp.

The vision impairment of AMD is a consequence of both scaring (i.e., fibrosis, fibrogenesis) and neovascularization. Because S1P is pro-angiogenic, it was reasoned that the anti-S1P mAb used in the experiments described in the previous examples would inhibit angiogenesis by reducing the survival, migration, and proliferation of endothelial cells (ECs); inhibit scar formation by reducing the survival, migration, and proliferation of fibroblasts; and inhibit the cross-talk between S1P with pro-angiogenic compounds including VEGF, bFGF, interleukins, and growth factors that contribute to uncontrolled vascularization during AMD. Thus, the uncontrolled proliferation of cells such as the ECs in AMD could be considered a hyperproliferative cell disorder.

Here, treatments consisted of intravitreal (IVR) injections of either the anti-S I P mAb or a non-specific isotype-matched mouse mAb. IVR injections consisted of 0.5ug of the anti-S1P mAb diluted into 2uL or an equal volume of vehicle. IVR injections were administered every 7 days starting 1 day prior to laser bums and lasting for the duration of the study. Just prior to IVR injections, mice were anesthetized with ketamine/xylene delivered IP. Under anesthesia the animal's eyes were moisturized frequently with normal saline. IVR injections were performed slowly into each animal's right eye with a 32 gauge needle. For all IVR injections, the eyes were covered with moisturizing Vaseline-containing standard antibiotics. The pupils of the mice were dilated with phenylephrine/atropine for 10 minutes and then anesthetized with ketamine/xyelene (5:1) for 5 minutes prior to inducing the laser bums. A cover slip was placed on the surface of the eye (lower side) with a clear ophthalmologic media to act as a lens for the laser. A light was shone into the eye to visualize the optical nerve and the neural retina. A fine laser was then focused onto the back of the retina and was set perpendicular to the back of the eye. Three burns were placed 1 optical disc (size of the optical nerve) away from the optical nerve between blood vessels (avoiding blood vessels). The settings for the laser were as follows: duration of 100mS, intensity of 250mW, and a diameter of 50 microns. The laser bums traveled through the neural retina and focused on the pigmentation of the RPE layer, causing a rupture of Bruch's membrane. Immediately after the bum, a pocket of fluid formed around the burn and marked the spot of the burn. The pocket resulted from fluid expanding from the heat of the burn. The pocket eventually diminished but a small burn spot could still be observed. Animals were observed until they fully recovered from the anesthesia.

Two weeks after rupture of Bruch's membrane, the animals were sacrificed and their eyes harvested and placed in paraformaldehyde overnight. The eyes were then washed in PBS and the RPE-choroid-sclera complex was isolated from the neural retina. The complex (~200microns in thickness) was then incubated in PBS containing Triton X-100 and an anti-glutanin-Rhodamine antibody overnight. The complex was then washed and flat mounted for evaluation. Using the Z-line imaging with confocal microscopy, 4 micron sections are imaged from the top to the bottom of the complex (~50 images). The central scar/vascularized area (- middle of the complex) was manually outlined and the images were independently analyzed for background levels of fluorescence. The background fluorescence was subtracted from the outlined area of each image and then each area was analyzed for relative fluorescence. The total fluorescence was then calculated. Each animal, with the 3 burns, was an n of 1.

The anti-S1P mAb significantly (p<0.05) reduced CNV lesion formation (~50% reduction) when administered via IVR injection, compared to IVR injection of a isotype matched non-specific monoclonal antibody.

### Example 14

### Fibrogenesis

Hyperproliferative disorders involving fibroblasts (i.e., fibrogenesis) include, but are not limited to, disorders of excessive scaring (i.e., fibrosis) such as age-related macular degeneration (AMD), cardiac remodeling and failure associated with myocardial infarction, excessive wound healing such as commonly occurs as a consequence of surgery or injury, keloids, and fibroid tumors. This Example 14 demonstrates that S1P is a potent activator of fibroblast proliferation, migration, and collagen gene expression in vitro and in vivo, and that an anti-S1P mAb is effective in reducing the S1P-mediated effects on fibroblast activity. In addition, the antibody was shown to mitigate scar formation in a cardiac model.

*In vitro* work with cultured fibroblasts demonstrated the potent ability of S1P to activate fibroblast proliferation, migration, and collagen gene expression. In these experiments, the anti-S I P mAb mitigated S1P-mediated effects and resulted in a diminution of fibroblast activity.

In order to demonstrate the beneficial effects of reducing fibroblast-mediated scar formation, an *in vivo* model of heart failure was developed by giving mice permanent coronary ligations during thoracotomy followed by a two-week take downs. In these studies, the anti-S1P mAb (25mg/kg) was administered via i.p. 48 hr. after the infarcts, followed by dosing every three days until termination of the study. 48 hr. post-infarct induction was chosen because it was reasoned that some scar formation was beneficial during this period and that the angiogenic effects of S1P would also be manifested immediately after the infarcts but would not necessarily be needed thereafter. Further, it was reasoned that excessive scar formation would be counterproductive after the 48 hr. period due to the profound maladaptive fibrosis that commonly results from the remodeling process. The increase in survivability of infarcted mice that were treated with the anti-S1P mAb and demonstrates that mitigating maladaptive cardiac fibrosis can result in improved survival has been demonstrated.

## Claims

1. A monoclonal antibody that binds sphingosine-1-phosphate (S-1-P) and inhibits S1P activity for use in a method of treating an S-1-P-associated hyperproliferative disorder in a mammal, wherein the S-1-P-associated hyperproliferative disorder is selected from the group consisting of a neoplasia, tumor angiogenesis, age- related macular degeneration, cardiac failure, inflammation and scarring.

2. The monoclonal antibody for use according to claim 1 wherein the neoplasia is a cancer.

3. The monoclonal antibody for use according to claim 2 wherein the cancer is a solid tumor or a hematological tumor.

4. The monoclonal antibody for use according to claim 1 wherein the mammal is selected from the group consisting of bovine, canine, equine, ovine, and porcine animals.

5. The monoclonal antibody for use according to to claim 1 wherein the mammal is human.

6. The monoclonal antibody for use according to claim 1, wherein the monoclonal antibody is contained in a composition further comprising a pharmaceutically acceptable carrier.

7. The monoclonal antibody for use according to claim 1 wherein the antibody is administered as a monotherapy.

8. The monoclonal antibody for use according to claim 1 wherein the antibody is administered as part of a combination therapy.

9. The monoclonal antibody for use according to claim 8 wherein the combination therapy, in addition to administration of the antibody, further comprises delivering a second therapeutic regimen selected from the group consisting of administration of a chemotherapeutic agent, radiation therapy, surgery, and a combination of any of the foregoing.

## Patentansprüche

1. Monoklonaler Antikörper, der Sphingosin-1-phosphat S-1-P) bindet und S1P Aktivität inhibiert, zur Verwendung in einem Verfahren zur Behandlung einer S-1-P assoziierten hyperproliferatorischen Störung in einem Säuger, wobei die S-1-P assoziierte hyperproliferatorische Störung aus der Gruppe ausgewählt wird, die aus einer Neoplasie, Tumorangiogenese, altersbedingter Makuladegeneration, Herzversagen, Entzündung und Vernarbung besteht.

2. Monoklonaler Antikörper zur Verwendung gemäß Anspruch 1, wobei die Neoplasie ein Krebs ist.

3. Monoklonaler Antikörper zur Verwendung gemäß Anspruch 2, wobei der Krebs ein solider Tumor oder ein hämatologischer Tumor ist.

4. Monoklonaler Antikörper zur Verwendung gemäß Anspruch 1, wobei der Säuger aus der Gruppe ausgewählt wird, die aus Rindern, Hunden, Pferden, Schafen und Schweinen besteht.

5. Monoklonaler Antikörper zur Verwendung gemäß Anspruch 1, wobei der Säuger ein Mensch ist.

6. Monoklonaler Antikörper zur Verwendung gemäß Anspruch 1, wobei der monoklonale Antikörper in einer Zusammensetzung enthalten ist, die ferner einen pharmazeutisch annehmbaren Träger umfasst.

7. Monoklonaler Antikörper zur Verwendung gemäß Anspruch 1, wobei der monoklonale Antikörper als Monotherapie verabreicht wird.

8. Monoklonaler Antikörper zur Verwendung gemäß Anspruch 1, wobei der monoklonale Antikörper als Teil einer Kombinationstherapie verabreicht wird.

9. Monoklonaler Antikörper zur Verwendung gemäß Anspruch 8, wobei die Kombinationstherapie, zusätzlich zu der Verabreichung des Antikörpers umfasst, ein zweites therapeutisches Regime zu erbringen, das aus der Gruppe ausgewählt wird, die aus der Verabreichung eines chemotherapeutischen Mittels, Strahlungstherapie, Operation und einer Kombination der vorstehend genannten ausgewählt wird.

## Revendications

1. Anticorps monoclonal qui se lie à sphingosine-1-phosphate (S-1-P) et inhibe l'activité de S-1-P à utiliser dans un procédé de traitement d'un trouble hyperprolifératif associé à S-1-P chez un mammifère, dans lequel le trouble hyperprolifératif associé à S-1-P est sélectionné parmi le groupe constitué de la néoplasie, l'angiogenèse tumorale, la dégénérescence maculaire liée à l'âge, l'insuffisance cardiaque, l'inflammation et la cicatrisation.

2. Anticorps monoclonal à utiliser selon la revendication 1, dans lequel la néoplasie est un cancer.

3. Anticorps monoclonal à utiliser selon la revendication 2, dans lequel le cancer est une tumeur solide ou une tumeur hématologique.

4. Anticorps monoclonal à utiliser selon la revendication 1, dans lequel le mammifère est sélectionné parmi le groupe constitué d'animaux bovins, canins, équins, ovins, et porcins.

5. Anticorps monoclonal à utiliser selon la revendication 1, dans lequel le mammifère est humain.

6. Anticorps monoclonal à utiliser selon la revendication 1, dans lequel l'anticorps monoclonal est contenu dans une composition comprenant en outre un support pharmaceutiquement acceptable.

7. Anticorps monoclonal à utiliser selon la revendication 1, dans lequel l'anticorps est administré comme monothérapie.

8. Anticorps monoclonal à utiliser selon la revendication 1, dans lequel l'anticorps est administré dans le cadre d'une polythérapie.

9. Anticorps monoclonal à utiliser selon la revendication 8, dans lequel la polythérapie, en plus de l'administration de l'anticorps, comprend en outre l'administration d'un deuxième schéma thérapeutique sélectionné parmi le groupe consistant à administrer un agent chimiothérapeutique, une thérapie par rayonnement, une chirurgie, et une combinaison de l'un quelconque des schémas précédents.
